# EUROPEAN PATENT APPLICATION

(11) **EP 2 143 807 A1**
(43) Date of publication of application: **13.01.2010**
(21) Application number: 09008333.8
(22) Date of filing: 25.06.2009
(51) Int. Cl.: C12Q 1/68

(54) **Method for methylation-selective amplification**

(30) Priority: 27.06.2008 EP 08159227; 25.03.2009 EP 09156169
(71) Applicant: Epigenomics AG, 10178 Berlin (DE)
(72) Inventor: Dietrich, Dimo, Dr., 10437 Berlin (DE)
(74) Representative: Hoppe, Georg Johannes

(57) **Abstract**

Aspects of the invention relate to a method for methylation selective amplification. The method comprising a DNA treatment, wherein cytosine is converted to uracil, uracil sulfonate or another base having a different binding behavior than cytosine, while methylated cytosine remains unchanged, and the amplification of treated DNA in the presence of at least one restriction enzyme, said enzyme digesting the amplification product derived either from converted methylated DNA or from converted unmethylated DNA during amplification. Aspects of the invention relate to a kit for performing the inventive method. Aspects of the invention relate also to the use of the inventive methods and kits.

## Description

The invention relates generally to novel methods for the methylation specific amplification. Particular embodiments relate generally to novel methods for quantification of methylated DNA or of unmethylated DNA.

### BACKGROUND

It is well known in the art that DNA as well as RNA can be methylated. The base 5-methylcytosine is the most frequent covalently modified base found in the DNA of eukaryotic cells. DNA methylation plays an important biological role in, for example, regulating transcription, genomic imprinting, and tumorigenesis (for review see, *e.g.*, Millar et al.: Five not four: History and significance of the fifth base; in The Epigenome, S. Beck and A. Olek (eds.), Wiley-VCH Publishers, Weinheim 2003, pp. 3-20). The identification of 5-methylcytosine is of particular interest in the area of cancer diagnosis. But the identification of methylation is difficult. Cytosine and 5-methylcytosine have the same base-pairing behavior, making 5-methylcytosine difficult to detect using particular standard methods. The conventional DNA analysis methods based on hybridization, for example, are not applicable. In addition, the methylation information is lost completely by the amplification by means of PCR.

Accordingly, current methods for DNA methylation analysis are based on two different approaches. The first approach utilizes methylation specific restriction enzymes to distinguish methylated DNA, based on methylation specific DNA cleavage. The second approach comprises selective chemical conversion (*e.g.*, bisulfite treatment; see e.g. WO 2005/038051) of unmethylated cytosines to uracil while methylated cytosines remain unchanged. Uracil has the same base pairing behavior as thymine. It therefore forms base pairs with adenine. Instead, 5-methylcytosine hybridizes with guanine still after bisulfite treatment. It is therewith possible to differentiate between methylated und unmethylated cytosines. Alernatively, cytosine may be converted by enzymes like for example cytidine-deaminase which converts unmethylated cytosine faster as methylated cytosine. An appropriate enzyme is described by Bransteitter et al. (Bransteitter et al.: "Activation-induced cytidine deaminase deaminates deoxycytidine on single-stranded DNA but requires the action of Rnase". PNAS 2003, 100(7): 4102-4107; WO 2005/005660)

The enzymatically or chemically pretreated DNA generated in these approaches is typically pre-amplified and analyzed in different ways (see, *e.g.,* WO 02/072880 pp. 1 ff; Fraga and Estella: DNA methylation: a profile of methods and applications; Biotechniques, 33:632, 634, 636-49, 2002). The pre-amplification of chemically or enzymatically pretreated DNA leads to an enhanced sensitivity of the subsequent detection reaction.

Currently several amplification methods are known to those skilled in the art that allow discrimination between methylated and unmethylated cytosines at pre-defined CpG positions. Some embodiments of these methods allow also a quantification of methylation i.e. the determination of the amount of DNA molecules that are methylated or unmethylated at said pre-defined CpG position within a mixture of DNA molecules.

Said methods are: restriction assay, primer extension, COBRA, MLA, MSP, MethyLight, HM, QM, and HQM.

*Restriction assay:* Several embodiments of the restriction assay method exist. According to these methods, in a first step, DNA is digested methylation-specifically. For this, either enzymes are used which digest DNA in case their recognition site is methylated or enzymes are used which digest DNA in case their recognition site is unmethylated. Subsequently, in a second step, DNA is amplified. Preferably, this amplification is a PCR, in particular a real-time PCR. The latter allows quantification of amplificates and therewith to deducing the amount of originally methylated or unmethylated cytosine at the position of interest. The principle of this method can be gathered for example from WO 2003/025215.

The restriction assay method has the disadvantage that it cannot be used for sensitive detection. The restriction assay method is based on a restriction enzymatic digestion followed by an amplification. Restriction enzymes normally do not cut all substrates (efficiency lower than 100%). If a restriction assay would be used for sensitive digestion, the background DNA would have to be digested, said background DNA being present in vast excess. Even if only a single copy of background DNA would remain, sensitive detection of the DNA of interest would be unfeasible. Also accidentally occurring single strands of background DNA would make sensitive detection impossible because single strands are indigestible for the usually applied restriction enzymes.

*Primer extension:* Several embodiments of the primer extension method exist. These methods are based on the conversion of unmethylated cytosines to uracil while methylated cytosines remain unchanged by means of bisulfite. The resulting converted DNA is then subjected to pre-amplification, in particular PCR. Thereafter a methylation specific elongation of a primer is carried out. The binding site of said primer is located up to 10 bases before the cytosine position to be analyzed, is adjacent to it, or encompasses it. After hybridization the primer is elongated by one up to 10 nucleotides. The said methods are for example described in US 6,251,594, WO 01/62961, WO 01/062960, and WO 01/062064.

COBRA (Combined Bisulfite Restriction Analysis): According to this method, bisulfite treated DNA is treated with bisulfite. After the treatment, the DNA is amplified by means of PCR. Thereafter, the amplified DNA is digested by means of restriction enzymes. Said enzymes are specific for bisulfite treated unmethylated sites. Digested or undigested fragments are finally detected for example by gel electrophoresis and in if the case may be hybridization of a specific labeled probe. Methylation levels in the original DNA sample are represented by the relative amounts of digested and undigested PCR product. The COBRA method is for example described in Xiong & Laird, Nucleic Acids Res. 25:2532-2534, 1997.

The COBRA method has also the disadvantage that it is not suitable for sensitive detection. According to the COBRA method, amplification occurs in a methylation unspecific way. Thereafter, the originally methylated DNA is digested. If the DNA of interest was originally only present in a very small amount, it is difficult to detect the digested DNA or the reduction of undigestion DNA e.g. in a gel.

*MLA (Methylation Sensitive Ligation and Amplification):* According to this method, DNA is subjected to a treatment wherein unmethylated cytosine is converted to uracil, while methylated cytosine remains unchanged. Preferably, said treatment is a treatment with bisulfite. Subsequently, the treated DNA is amplified by means of at least two pairs of essentially complementary probe oligonucleotides. The methylation status of the cytosine of interest in the original DNA is deduced from the presence or absence of amplificates. The MLA method is for example described in WO 03/057909.

*MSP (Methylation Specific Polymerase Chain Reaction):* According to this method, DNA is treated with bisulfite resulting in a conversion of unmethylated cytosines to uracil while methylated cytosines remain unaltered. After said conversion, the DNA is subjected to PCR amplification by means of methylation-specific primers. The MSP method is for example described in Herman et al.: Methylation specific PCR: a novel PCR assay for methylation status of CpG islands. Proc Natl Acad Sci U S A. 93:9821-6, 1996. When carried out as a real-time variant, the MSP allows a quantification of amplified products. Thus, it is possible to deduce the amount of subjected DNA being methylated or unmethylated at the cytosine positions of interest.

*MethyLightmethod:* According to this method, DNA is treated with a modifying agent wherein unmethylated cytosines become modified. Thereafter the treated DNA is analyzed by real-time PCR with one or more probe. The binding site of said probe or probes comprises one or more CpG positions. Thus, the amount of amplification product derived either from DNA with methylated CpG positions or from DNA with unmethylated CpG positions is detected during amplification. From this, the amount of original DNA being methylated or unmethylated at the analyzed CpG positions is deduced. Suitable probe systems for analysis are Taqman probe, LightCycler probes or Scorpion primers. The MethyLight method is for example described in WO 00/70090; US 6,331,393; or Trinh et al.: DNA methylation analysis by MethyLight technology. Methods, 25:456-62, 2001).

*HM (HeavyMethyl) method:* According to this method, in a first step, unmethylated cytosines of a DNA are converted to uracil while methylated cytosines remain unchanged. The converted DNA is then subjected to an amplification reaction. The amplification of the converted methylated or unmethylated DNA of interest is detected during the amplification while undesired background DNA is simultaneously suppressed by means of a blocker. Said blocker is either specific for background converted methylated DNA or background converted unmethylated DNA. Preferably, the HM method is carried out as a real time variant. The HM method is described for example in WO 02/072880.

*QM (Quantitative Methylation) method:* According to this method, DNA is subjected to treatment, wherein methylated cytosine remains unchanged and unmethylated cytosine is converted to uracil or another base having different base pairing behavior than cytosine. The converted DNA is then subjected to a real time PCR amplification in the presence of two probe systems. One probe system is specific for the case of methylation of the CpG positions of interest. The other probe system is specific for the case that the CpG positions of interest are not methylated. Suitable probe systems are for example Taqman probes or LightCycler probes. In case of Taqman probes, each probes system consists of one probe. In case in of Lightcycler probes, each probe system consists of two probes acting cooperatively. By means of the said probe systems, the amplification products derived from correspondent converted methylated or unmethylated DNA are detected during amplification. From this, the amount of original DNA being methylated or being unmethylated is deduced. The QM method is for example described in WO 2005/098035.

*HQM (Heavy Quantitative Methylation) method:* According to this method, unmethylated cytosines of a double stranded DNA are converted to uracil or a base having a different base pairing behavior than cytosine while methylated cytosine remains unchanged. In preferred embodiments of the HQM method both converted strands are subjected to methylation analysis, in particular wherein originally reverse complementary regions are analyzed in the subjected DNA. In particular preferred is the analysis of both converted DNA strands in methylation specific reactions. Such reactions may be: PCR, isothermal amplification, LCR, MSP, HeavyMethyl, bisulfite sequencing, microarrays, methylation specific restriction enzymes, real-time PCR, HeavyMethyl, MethyLight, QM, methylation sensitive primer extension. The HQM method is descirbed in detail in WO 2008/017411

A drawback of the known methods is that sensitivity and specificity suffer if only single CpG positions are being analyzed.

### DETAILED DESCRIPTION OF ASPECTS OF THE INVENTION

For achieving various technical objects, particular aspects of the invention teach and provide a method for methylation-selective amplification. The method comprises (a) the treatment of a DNA sample, wherein cytosine is converted to uracil, uracil sulfonate or another base having a different binding behavior than cytosine, while methylated cytosine remains unchanged; and (b) the amplification of the treated DNA by means of at least one oligonucleotide in the presence of at least one restriction enzyme. Said restriction enzyme digests the amplification product derived either from converted methylated DNA or from converted unmethylated DNA during amplification. Wherein the amplification product derived from converted methylated DNA is digested, the amplification product derived from unmethylated DNA is further amplified and detected. Wherein the amplification product derived from converted unmethylated DNA is digested, the amplification product derived from methylated DNA is further amplified and detected.

Particular aspects of the invention teach and provide a method for methylation-selective amplification characterized by high sensitivity. This is achieved by digesting the amplification product generated in an amplification cycle at the end of each amplification cycle and/or during amplification.

Particular aspects of the invention teach and provide a method for the sensitive detection of methylated or unmethylated DNA. Said aspects comprise the methylation-selective amplification according to the invention and the quantification of amplification products during amplification and/or at the end of the amplification reaction.

Particular aspects of the invention teach a kit for methylation-selective amplification and/or for sensitive detection of methylated or unmethylated DNA.

Particular aspects of the invention teach the use of the herein taught and provided methods as well as the herein taught kits.

### Advantages of aspects of the invention.

Particular aspects of the invention are **characterized in that** they have pronounced advantages over the known state of the art methods of methylation-selective amplification or sensitive detection of methylated or unmethylated DNA. The disadvantages of these state of the art methods have already been described above.

In this respect, particular aspects of the invention are characterized by the following advantages:

Particular aspects of the invention are characterized by high sensitivity. According to the invention this high sensitivity is achieved by repetitive digestion of amplification product during amplification or at the end of each amplification step and/or during amplification. According to the invention, the amplification step comprises the extension of at least one oligonucleotide. Through this double stranded DNA is generated that is able to be digested by restriction enzymes. The actual digestion takes place during the extension reaction, however may also take place at any other phase of the amplification. Additionally, the inventive high sensitivity is enabled by amplification.

Moreover, particular aspects of the invention are characterized by high specificity. According to the invention this high specificity is achieved by specificity of the at least one oligonucleotide used for amplification; by the specificity of the one or more used restriction enzymes; and by repetitive removal of undesired amplification product during amplification. Through the later only the amplification product of interest remains for further amplification and detection.

As described above, so far known method for methylation-selection amplification have either a lower sensitivity or require more than one CpG position is order to achieve a comparable specificity. For example, the MSP- method requires two regions comprising CpG positions that are co-methylated i.e. said CpG-positions are either all methylated or are all unmethylated. In case no second co-methylated region exist, the second MSP-primer is not able to bind, thus no amplification is possible.

In addition, all of the state of the art methods have the disadvantage that the actual assays require a certain architecture. For example, the MSP method requires two primer binding sites wherein each binding site comprises CpG positions, the CpG positions of both binding site have to be co-methylated. In addition, the primer binding sites can not be chosen arbitrarily. Instead they have to be chosen so that only a site-specific amplification is possible. This is in particularly complicated by the fact that a bisulfite conversion reduces the complexity of DNA from a four base alphabet to a three base alphabet. However, according to the invention, no such special architecture is necessary. According to the invention, the primers for amplification can be chosen arbitrarily. The only requirement is that one primer is located at or 5' from the CpG position interest while the other primer is located at or 3' from the CpG position of interest. This requirement can easily be fulfilled. The methylation specificity according to the invention is achieved by the numerous restriction enzymes that digest amplification product derived either from methylated DNA or from unmethylated DNA.

A further advantage of the inventive method is that it is very sensitive to the methylation status of an individual CpG position. The instant a CpG position is methylated and a corresponding restriction enzyme is used that digests amplification product derived from unmethylated DNA, only the said methylated DNA after treatment is amplified and detected. The same also holds true for the opposite case. The instant a CpG position is unmethylated and a corresponding restriction enzyme is used that digests amplification product derived from methylated DNA, only the said unmethylated DNA after treatment is amplified and detected. However state of the art methods are as a general rule not so sensitive. For example, the MSP method requires several CpG positions all of them being co-methylated. However, if one of them is not co-methylated with the other positions, still an erroneous amplification occurs.

### Method of aspects of the invention.

Aspects of the present invention relate to a method for methylation-selective amplification. The method comprises the conversion of cytosines of a DNA to uracil, uracil-sulfonate or another base having a different binding behavior than cytosine. Thereafter, the DNA is amplified, wherein undesired amplification product is removed during amplification. Preferably, this is achieved by restriction enzyme digestion. For this, one or more restriction enzymes cut only amplification product that is derived in case of methylation of the CpG position of interest. Alternatively, for this, one or more restriction enzymes cut only amplification product that is derived in case of non-methylation of said CpG position of interest. Each of the said one or more enzymes have at a recognition site that comprise the position of the cytosine being able to be methylated. The actual restriction site of each of the said enzymes may either comprise the position of interest or not.

Preferably, an amplification product does comprise only one restriction site of said enzyme. Preferably, an amplification product does comprise 2, 3, 4, or more restriction sites of said one or more enzymes.

Preferably, 1, 2, 3, 4, 5, 10, 15, 20 or more CpG positions are simultaneously analyzed in a DNA sample. Preferably, for every CpG position two amplification products arise from the extension of at least one applied oligonculeotide.

Aspects of the present invention relate to a method for methylation-selective amplification, comprising (a) subjecting a DNA sample to a treatment, wherein cytosine is converted to uracil, uracil sulfonate or another base having a different binding behavior than cytosine, while methylated cytosine remains unchanged; and (b) amplifying the treated DNA by means of at least one oligonucleotide in the presence of at least one restriction enzyme, said enzyme digesting amplification product derived either from converted methylated DNA or from converted unmethylated DNA during amplification.

The term "amplification product" specifies the double stranded DNA generated by the extension of one of the applied at least one oligonucleotide. The applied enzyme, although not necessarily 100% active during the entire amplification, is at least able to digest the amplification product sufficiently during amplification. Preferably, in order to allow a more efficient digestion of amplification product, an additional step may be inserted into the amplification reaction ensuring the efficient digestion of amplification product. For this, a suitable temperature has to be chosen, on one hand enabling an efficient digestion of amplification product, on the other hand minimizing unspecific amplification. According to preferred embodiment, a suitable temperature for digestion is selected from the range of 30-80°C, preferably from the range of 37-73°C, and most preferably from the range of 55-68°C. The person skilled in the art is able to identify the best suitable temperature by routine testing.

In particular, one of the following enzymes is preferred, wherein said temperature for extension and digestion is selected from the range of 55-70°C: Tsp509l, BstBl, BstUl, and Tfil.

According to aspects of the invention, amplification product is preferentially digested during the extension of one of the applied one or more oligonucleotides. However it may also be possible according to the invention that amplification product is digested during any other phase of the amplification.

According to a preferred embodiment of the invention, the inventive method for methylation-selective amplification is multiplexed. Thus, simultaneously several CpG positions of interest are analysed. For this, in a first step, a DNA sample is subjected to a treatment, wherein cytosine is converted to uracil, uracil sulfonate or another base having a different binding behavior than cytosine, while methylated cytosine remains unchanged. In a second step, the treated DNA is amplified, wherein, at least in the first cycle, two amplification products may be generated per CpG position of interest. One amplification product is derived in case of methylation of the position, while the other is derived in case of non-methylation of said position. During amplification one of the said two amplification products for each CpG position is digested. Thus, only one of the said two amplification products for each CpG positon will be amplified, while the other is repetitively removed. Thus, during and at the end of amplification one of the two amplification products is predominantly detectable for every CpG position of interest. For digestion of the undesired amplification products, one or more suitable restriction enzymes are applicable.

According to a preferred embodiment the at least one restriction enzyme is selected from the group consisting of Tsp5091, Acll, BstBi, BstUl, BstZ17l, CviQl, Hpal, and Tfil. Also preferred are isochizomers of said enzymes.

In a preferred embodiment an enzyme is applied that cuts only amplification product corresponding to an unmethylated CpG position of interest in the DNA subjected to the method of the invention. Thus, said enzyme enabling a selective amplification and therewith a selective detection of methylated DNA. In a particular preferred embodiment, said enzyme is Tsp509l.

In a preferred embodiment an enzyme is applied that cuts only amplification product corresponding to a methylated CpG position of interest in the DNA subjected to the method of the invention. Thus, said enzyme enabling a selective amplification and therewith a selective detection of unmethylated DNA. In a particular preferred embodiment, said enzyme is BstBl, BstUl, BstZ17l, CviQl, Hpal, Tfil, or combinations of the said.

According to a preferred embodiment, one of said restriction enzymes is an enzyme said is suitable for digesting amplification product derived from unmethylated DNA, said enzyme having a recognition sequence that (i) has a 5' terminal A and does not comprise a G; (ii) has a 3' terminal T and does not comprise a C; (iii) or both.

In a preferred embodiment, an restriction enzyme is used that digests amplification product derived from unmethylated DNA. The recognition sequence of said restriction enzyme is **characterized in that** (i) its most 5' located nucleotide is A and it has no G; (ii) its most 3' located nucleotide is T and it has no C; (iii) its most 5' located nucleotide is A, its most 3' located nucleotide is T, and it has neither a G nor a C.

According to a preferred embodiment, one of said restriction enzymes is an enzyme said is suitable for digesting amplification product derived from methylated DNA, said enzyme having a recognition sequence that comprises a cytosine of a CpG position.

In a preferred embodiment, a restriction enzyme is used that digests amplification product derived from unmethylated DNA. The recognition sequence of said restriction enzyme is **characterized in that** it comprises a cytosine that is part of a CpG position (CpG dinucleotide). The G of said CpG position is either part of the recognition sequence or not.

According to a preferred embodiment, the amplification product derived from converted unmethylated DNA comprise each at least one binding site (A) of said one or more oligonucleotides flanking an extended segment (B), wherein
- (A) and/or (B) comprise at least one sequence that either consist of the recognition sequence of the used enzyme and thereto an immediately 3' located G, or consist of the recognition sequence of the used enzyme and thereto an immediately 5' located C; and wherein
- (B) does not comprise a sequence being selected from the group consisting of recognition sequence of the used enzyme and thereto an immediately 3' located T, recognition sequence of the used enzyme and thereto an immediately 3' located A, recognition sequence of the used enzyme and thereto an immediately 3' located C, recognition sequence of the used enzyme and thereto an immediately 5' located G, recognition sequence of the used enzyme and thereto an immediately 5' located T, and recognition sequence of the used enzyme and thereto an immediately 5' located A.

In a preferred embodiment, an inventive amplification product has at least one binding site (A) for one or more oligonucleotides to initiate extension and an adjacently located extension region (B). The amplification product derived from unmethylated DNA is **characterized in that** (A), (B) or both comprise at least one sequence selected from the following: recognition sequence of the used enzyme being flanked on its 3' side by G, recognition sequence of the used enzyme being flanked on its 5' side by C. In addition the amplification product derived from unmethylated DNA is **characterized in that** (B) does not comprise a sequence selected from the following: recognition sequence of the used enzyme being flanked on its 3' side by T, recognition sequence of the used enzyme being flanked on its 3' side by C, recognition sequence of the used enzyme being flanked on its 3' side by A, recognition sequence of the used enzyme being flanked on its 5' side by G, recognition sequence of the used enzyme being flanked on its 5' side by T, recognition sequence of the used enzyme being flanked on its 5' side by A.

According to a preferred embodiment, region (A) of amplification product derived from methylated DNA comprises at least one sequence being selected from the group consisting of recognition sequence of the used enzyme and thereto an immediately 3' located T, recognition sequence of the used enzyme and thereto an immediately 3' located A, recognition sequence of the used enzyme and thereto an immediately 3' located C, recognition sequence of the used enzyme and thereto an immediately 5' located G, recognition sequence of the used enzyme and thereto an immediately 5' located T, and recognition sequence of the used enzyme and thereto an immediately 5' located A; and the treated DNA is amplified by means of at least one modified oligonucleotide.

In a prefered embodiment, the at least one binding site (A) of amplification product derived from methylated DNA comprises at least one sequence selected from the following: recognition sequence of the used enzyme being flanked on its 3' side by T, recognition sequence of the used enzyme being flanked on its 3' side by C, recognition sequence of the used enzyme being flanked on its 3' side by A, recognition sequence of the used enzyme being flanked on its 5' side by G, recognition sequence of the used enzyme being flanked on its 5' side by T, recognition sequence of the used enzyme being flanked on its 5' side by A. In addition, said preferred embodiment is **characterized in that** DNA after treatment is amplified by means of one or more modified oligonucleotides as specified herein.

According to a preferred embodiment, the amplification product derived from treated methylated DNA comprise each at least one binding site (A) of said one or more oligonucleotides flanking an extended segment (B), wherein (A) and/or (B) comprise at least one recognition sequence of the used enzyme.

According to a particular preferred embodiment, (i) one of said restriction enzymes is Tsp5091; and (ii) the amplification product derived from converted methylated DNA and the amplification product derived from converted unmethylated DNA comprise each at least one binding site (A) of said one or more oligonucleotides flanking an extended segment (B), wherein
- (A) and/or (B) comprise at least one sequence that corresponds to a sequence of a untreated DNA strand, said sequence being selected from the group consisting of AATCG, AACCG, CGATT, and CGGTT; and wherein
- (B) does not comprise a sequence corresponding to a sequence of an untreated DNA strand, said sequence being selected from the group consisting of AATT, AACT, AATCA, AATCT, AATCC, AACCA, AACCT, AACCC, AGTT, AATT, AGGTT, TGGTT, GGGTT, AGATT, TGATT, and GGATT.

In a particular preferred embodiment, an amplification product digesting enzyme is Tsp5091. In general, an amplification product comprises regions (A) and (B) being located adjacent to each other. While the one or more oligonucleotides becoming extended are reverse complementary to (A), (B) is the region being extended. According to said particular preferred embodiment, region (A), (B), or both alone or together comprise the sequence AATTG. In other words the sections corresponding to regions (A) and (B) of the originally DNA subjected to the inventive method comprises alone or together at least one of the following sequences: AATCG, AACCG, CGATT, CGGTT. In addition, according to said particular preferred embodiment, the section corresponding to region (B) of the originally DNA subjected to the inventive method does not comprise one of the following sequences: AATT, AACT, AATCA, AATCT, AATCC, AACCA, AACCT, AACCC, AGTT, AATT, AGGTT, TGGTT, GGGTT, AGATT, TGATT, GGATT.

In a particular preferred embodiment, an amplification product comprises two regions (A) that flank both regions (B). Thus the regions (A) are located upstream and downstream of (B).

According to a particular preferred embodiment, (i) (A) comprises at least one sequence that corresponds to a sequence of an untreated DNA strand, said sequence being selected from the group consisting of AATT, AACT, AATCA, AATCT, AATCC, AACCA, AACCT, AACCC, AGTT, AATT, AGGTT, TGGTT, GGGTT, AGATT, TGATT, and GGATT; and wherein (ii) the treated DNA is amplified by means of at least one modified oligonucleotide.

In a particular preferred embodiment, the section corresponding to region (A) of the originally DNA subjected to the inventive method comprises one of the following sequences AATT, AACT, AATCA, AATCT, AATCC, AACCA, AACCT, AACCC, AGTT, AATT, AGGTT, TGGTT, GGGTT, AGATT, TGATT, GGATT. In addition said particular preferred embodiment comprises one or more modified oligonucleotides. Said modified oligonucleotides bind to said sequences after treatment and prevent a digestion of amplification product at corresponding sites.

According to the invention, said modified oligonucleotide is any oligonucleotide that is able to prevent the restriction enzyme digestion of amplification product at region (A), said region (A) comprising a restriction site of said enzyme, and said restriction site corresponding not to a CpG position. Preferably, the backbone of the oligonucleotide is at least in parts modified whereupon the restriction site of the applied enzyme in the amplification product becomes un-detachable for said enzyme. In a particular preferred embodiment, said oligonucleotide is modified by uracil, inosine, and/or abasic site, whereupon the applied enzyme is not able to recognize the site in region (A) that corresponds to a restriction site of said enzyme in the DNA after treatment, said DNA being applied to amplification. In particular preferred, said oligonucleotide is modified by one or more bases that form non Watson-Crick base pairings, whereupon the applied enzyme is not able to recognize the site in region (A) that corresponds to a restriction site of said enzyme in the DNA after treatment, said DNA being applied to amplification. In particular preferred are oligonucleotides that comprise two or more of said modifications.

According to a particular preferred embodiment, one of said restriction enzymes is Acll or BstBl and the amplification product comprises at least one sequence that corresponds to the sequence AACGTT of an untreated DNA strand.

In a particular preferred embodiment, an amplification product digesting enzyme is Acll, BstBl, or both. Accordingly, DNA subjected to the method of the invention comprises at least once the sequence AACGTT.

According to a particular preferred embodiment, one of said restriction enzymes is BstUl and the amplification product comprises at least one sequence that corresponds to the sequence CGCG of an untreated DNA strand.

In a particular preferred embodiment, an amplification product digesting enzyme is BstUl. Accordingly, DNA subjected to the method of the invention comprises at least once the sequence CGCG.

According to a particular preferred embodiment, one of said restriction enzymes is BstZ171 and the amplification product comprises at least one sequence that corresponds to the sequence GTATAC of an untreated DNA strand.

In a particular preferred embodiment, an amplification product digesting enzyme is BstZ171. Accordingly, DNA subjected to the method of the invention comprises at least once the sequence GTATAC.

According to a particular preferred embodiment, one of said restriction enzymes is CviQl and the amplification product comprises at least one sequence that corresponds to the sequence GTAC of an untreated DNA strand.

In a particular preferred embodiment, an amplification product digesting enzyme is CviQl. Accordingly, DNA subjected to the method of the invention comprises at least once the sequence GTAC.

According to a particular preferred embodiment, one of said restriction enzymes is Hpal and the amplification product comprises at least one sequence that corresponds to the sequence GTTAAC of an untreated DNA strand.

In a particular preferred embodiment, an amplification product digesting enzyme is Hpal. Accordingly, DNA subjected to the method of the invention comprises at least once the sequence GTTAAC.

According to a particular preferred embodiment, one of said restriction enzymes is Tfil and the amplification product comprises at least one sequence that corresponds to the sequence GAWTC of an untreated DNA strand.

In a particular preferred embodiment, an amplification product digesting enzyme is Tfil. Accordingly, DNA subjected to the method of the invention comprises at least once the sequence GAWTC. As it is known to a person skilled in the art, the variable "W" stands for A or T/U.

According to a preferred embodiment the treatment, wherein cytosine is converted to uracil, uracil sulfonate, or another base having a different binding behaviour than cytosine, while methylated cytosine remains unchanged, is a treatment with a reagent, a bisulfite, an enzyme, or a cytidine deaminase.

In a preferred embodiment, the original sample DNA subjected to the method of the invention is treated with a reagent or enzyme. Preferably, said reagent is a bisulfite. Preferably, said enzyme is a cytidine deaminase. Numerous kinds of such treatments are known to those skilled in the art. However particularly preferred are those treatments as described in PCT/DE2008/000958 page 15 line 23 - page 20 line 34 as filed (international application number PCT/IB2008/002118, publication number WO 2008/149237). Furthermore, treatments are also preferred as described in US 2006-0134643 (US serial number 10/964,167) on page 6 line 1 - page 7 line 10 and page 8 line 9 - page 14 line 5.

According to a preferred embodiment of the current invention, the amplification reaction is a PCR, real-time PCR, LCR, real-time LCR, primer extension, or isothermal amplification.

In a preferred embodiment, after the treatment of DNA, wherein cytosine is converted to uracil, uracil sulfonate or another base having a different binding behavior than cytosine, while methylated cytosine remains unchanged, said treated DNA is amplified. Preferably, said treated DNA is amplified by means of PCR, LCR, primer extension or isothermal amplification. In particular, said treated DNA is amplified by means of PCR.

In a preferred embodiment, amplification of treated DNA is carried out as a real time variant. In particular, said treated DNA is amplified by means of real-time PCR.

In a preferred embodiment, amplification of treated DNA comprises at least one of the group consisting of primer, blocker, probe, polymerase, ligase. According to the invention, a primer may be any oligonucleotide or oligomer suitable for initiating amplification. According to the invention, a blocker may be any oligonucleotide or oligomer suitable for preventing amplification. According to the invention, a probe may be any oligonucleotide or oligomer suitable for detecting an amplification product. According to the invention, said polymerase is a heatstable polymerase. According to the invention, said ligase is a heat-stable ligase.

In a preferred embodiment, the amplification of treated DNA comprises additionally the features of at least one of the following methods: restriction assay, primer extension, COBRA, MLA, MSP, MethyLight, HM, QM, HQM.

In a preferred embodiment comprising additional features of the restriction assay method, DNA is subjected to methylation sensitive restriction enzyme digestion prior the treatment in which cytosine in converted to uracil, uracil-sulfonate or another base having a different base pairing behaviour than cytosine, while methylated cytosine remains unchanged.

In a preferred embodiment comprising additional features of the primer extension method, at least one primer is used for amplification of treated DNA, wherein said primer either (a) hybridizes to treated methylated DNA or to treated unmethylated DNA, or (b) is extended depending on the methylation status of one or more CpG positions of interest.

In a preferred embodiment comprising additional features of the COBRA method, amplification product is digested by restriction enzymes after the amplification is completed.

In a preferred embodiment comprising additional features of the MLA method, two pairs of oligonucleotides are applied for amplification, wherein the two pairs are reverse complementary to each other and wherein the 3' terminal end of one oligonucleotide of a pairs is located in close proximity to the 5' terminal end of the other oligonucleotide of the same pair. Preferably, the oligonucleotides are separated by a distance of less than 20 nucleotides, preferably less than 15 nucleotides, more preferably less than 10 nucleotides, and most prefereably the two oligonucleotides are located immediately adjacent to each other.

In a preferred embodiment comprising additional features of the MSP method, at least one primer is used for amplification of treated DNA that is able to differentiate between treated methylated DNA and treated unmethylated DNA. Preferably but not limited to, said embodiment is carried out as real time variant.

In a preferred embodiment comprising additional features of the MethyLight method, at least one probe suitable for detecting an amplification product is used. Preferably but not limited to, said embodiment is carried out as real time variant.

In a preferred embodiment comprising additional features of the HeavyMethyl method, at least one blocker is used, said blocker is suitable for preventing amplification of treated methylated DNA or of treated unmethylated DNA. Preferably but not limited to, said embodiment is carried out as real time variant.

In a preferred embodiment comprising additional features of the QM method, two probes are used, wherein one probe is suitable for detecting amplification of treated methylated DNA and wherein the other probe is suitable for detecting amplification of treated unmethylated DNA. Preferably but not limited to, said embodiment is carried out as real time variant.

In a preferred embodiment comprising additional features of the HQM method, double stranded DNA is subjected to the inventive treatment wherein unmethylated cytosine is converted while methylated cytosine remains unchanged. In addition, both strand of said DNA after treatment are methylation specifically analyzed.

According to a preferred embodiment, undigested amplification product is detected by means of ethidium bromide, SYBR green, probe, oligonucleotide probe, PNA probe, Taqman probe, Lightcycler probe, scorpion primer, RNAenzyme, or DNAenzyme.

In a preferred embodiment, undigested amplification product is detected during amplification and/or at the end of amplification. Preferably, the amplification is detected during amplification, in particular wherein amplification is a real-time PCR amplification.

In a preferred embodiment, undigested amplification product is detected by any means known in the art. Preferably, undigested amplification product is detected by means of ethidium bromid after amplification. Also preferred is the detection by means of SYBR green or probe during amplification or after amplification. In particular preferred is the detection during amplification. For this, preferably, a probe, scorpion primer or enzyme is used. However and herewith also preferred are other means known in the art. A probe is any oligonucleotide or oligomer suitable for detecting an amplification product. A scorpion primer is an oligonucleotide or oligomer comprising two regions. One region is suitable for initiating amplification, while the other region is suitable for detecting the amplification product. Scorpion primers and their use in methylation analysis are described in WO 2005/024056. Preferably, an enzyme is a Ribozyme or a DNAzyme. Suitable Ribozymes or DNAzymes and their use in methylation analysis are described in EP 1882746.

A particular preferred embodiment comprises additionally quantifying the undigested amplification product during the amplification or thereafter.

In a particular preferred embodiment, undigested amplification product is quantified during amplification or thereafter. A person skilled in the art is in knowledge of suitable ways of quantification. In particular preferred is the quantification by real time amplification, most preferably by means of real-time PCR. The quantification of amplification product during amplification and thus the quantification of applied template i.e. treated methylated or unmethylated DNA by means of real-time PCR is also known as threshold-value analysis.

According to threshold-value analysis, the quantity of amplification product is determined in the exponential phase of the amplification, rather than at the end of the amplification. Such threshold, real-time methods presume that the amplification efficiency is constant in the exponential phase. The art-recognized threshold value 'CT' is a measure corresponding, within a PCR reaction, to the first PCR cycle in which the signal in the exponential phase of the amplification is greater than the background signal. Absolute quantification is then determined by means of a comparison of the CT value of the investigated (test) DNA with the CT values of calibration points of a standard curve. However and herewith also preferred are other variants of threshold-value analysis as known to those skilled in the art. An overview of real time PCR based quantification can be obtained from WO 2005/098035; Real-Time PCR: An Essential Guide, Horizon Bioscience, Kirstin Edwards, Julie Logan and Nick Saunders, May 2004 ISBN: 0-9545232-7X; Real-time PCR, M. Tevfik Dorak, Taylor & Francis, April 2006), ISBN: 041537734X; Mackay IM, Arden KE, Nitsche A. Real-time PCR in virology. Nucleic Acids Res. 2002 Mar 15;30(6):1292-305; Bernard PS, Wittwer CT. Real-time PCR technology for cancer diagnostics. Clin Chem. 2002 Aug;48(8):1178-85; Bernhard Kaltenboeck and Chengming Wang. Advances in real-time PCR: Application to clinical laboratory diagnostics. Advances in Clinical Cancer, 2005; 40:219-259.

Said embodiment comprising quantification of amplification product during amplification by means of real-time PCR is in particular useful for the sensitive detection of a methylated or unmethylated CpG position.

Quantification of the degree of methylation at one or more CpG positions is required in many applications including, but not limited to, classification of tumors, obtaining diagnostic information, obtaining prognostic information, monitoring, or for predicting drug effects/responses.

According to a particular preferred embodiment, amplification product derived from treated unmethylated DNA is digested during amplification and the amount of methylated DNA in the DNA subjected into the inventive method is deduced from the results of the quantification of undigested amplification product. Said undigested amplification product is derived from treated methylated DNA.

According to a particular preferred embodiment, amplification product derived from treated methylated DNA is digested during amplification and the amount of unmethylated DNA in the DNA subjected into the inventive method is deduced from the results of the quantification of undigested amplification product. Said undigested amplification product is derived from treated unmethylated DNA.

According to a preferred embodiment, DNA is isolated from tissue or body fluids. Preferably, DNA is isolated from biopsy or tissue section. Thereby the term "biopsy" refers to any kind of needle biopsy or any kind of tissue sample collected during a surgery. Moreover, the term "tissue section" refers to any part of a biopsy for example derived by microtom sectioning of the biopsy. Preferably, tissue or biopsy samples are unfixed. Preferably, they are embedded into paraffin or comparable compounds. Preferably, they are fixed with reagents for fixation, in particuar formalin. Preferably, DNA is isolated from a body fluid sample selected from the group consisting of blood sample, plasma sample, serum sample, body fluid sample, saliva sample, urine sample, semen sample, sample of the fluid from the pleural cavity, sample from the fluid from the peritoneal cavity, sample of the cerebrospinal fluid, smear from a epithelial surface, sputum sample, stool sample, ejaculate sample, tears sample, sweat sample, lymph fluid sample, bronchial lavage sample, pleural effusion sample, meningal fluid sample, glandular fluid sample, fine needle aspirates sample, nipple aspirates fluid sample, spinal fluid sample, conjunctival fluid sample, vaginal fluid sample, duodenal fluid sample, prancreatic juice sample, or bile sample.

According to a preferred embodiment, DNA is isolated according to standard methods known to those skilled in the art. Preferably, DNA is isolated by means of beads, silica beads, plastic beads, membranes, silica membrane, ultrafiltration, Microcon filter device, gel filtration, filter device, silica surface, silica membrane, magnetic particle, polystyrol particle, polystyrol surface, positively charged surface, and positively charged membrane, charged membrane, charged surface, charged switch membrane, or charged switched surface. In particular preferred is the DNA isolation from a sample by means of SiMAG/FK-Silanol beads (Chemicell GmbH, Germany, Article Number 1101-01 or 1101-05) or beads of the chemagic Viral DNA/RNA Kit special in a previous step (Chemagen Biopolymer-Technologie Aktiengesellschaft; Article Number 1002).

### Kits of aspects of the invention

Aspects of the present invention relate to a kit, comprising
(a) a bisulfite or a cytidin-deaminase;
(b) at least one oligonucleotide or oligomer usable as a primer;
(c) a polymerase and/or a ligase;
(d) at least one enzyme of the group consisting of Tsp5091, Acll, BstBl, BstUl, BstZ17l, CviQl, Hpal, and Tfil; and
(e) optionally, at least one selected from the group consisting of ethidium bromide, SYBR green, probe, oligonucleotide probe, PNA probe, Taqman probe, Lightcycler probe, scorpion primer, RNAenzyme, DNAenzyme, blocker, blocker oligonucleotide, blocker PNA oligomer.

Aspects of the present invention relate also to a kit for the methylation selective amplification and for the sensitive detection of methylated DNA or unmethylated DNA, respectively. Said kit comprises
(a) a bisulfite or a cytidin-deaminase;
(b) at least one oligonucleotide or oligomer usable as a primer;
(c) a polymerase and/or a ligase;
(d) at least one enzyme of the group consisting of Tsp509l, Acll, BstBl, BstUl, BstZ17l, CviQl, Hpal, and Tfil.

Preferably, said kit further comprises
(e) at least one selected from the group consisting of ethidium bromide, SYBR green, probe, oligonucleotide probe, PNA probe, Taqman probe, Lightcycler probe, scorpion primer, RNAenzyme, DNAenzyme, blocker, blocker oligonucleotide, blocker PNA oligomer.

Preferably, said bisulfite is sodium bisulfite. Preferably said polymerase is a heat stable polymerase. Preferably, said ligase is a heat stable ligase. Preferably, Tsp5091 is used as enzyme for the methylation-selective amplification and the sensitive detection of methylated DNA. Preferably, Acll, BstBl, BstUl, BstZ17l, CviQl, Hpal, or Tfil is used as enzyme for the methylation-selective amplification and the sensitive detection of unmethylated DNA.

In a preferred embodiment, the inventive kit also comprises suitable buffers and reagents for amplification and/or digestion. Preferably, these buffers and reagents are known buffers and reagents suitable for amplification. Preferably, said buffers and reagents are adjusted to improve the performance of applied restriction enzyme. A person skilled in the art may obtain such buffers and reagents by routine testing.

In a preferred embodiment, the inventive kit also comprises buffers, reagents, or devices for performing a treatment with bisulfite, in particular with sodium bisulfite. Preferably, the inventive kit comprises one or more ultrafiltration devices or one more gelfiltration devices. Preferably, the inventive kit comprises DNA binding or retaining devices that are useful in purifying DNA treated with bisulfite. Such DNA binding or retaining devices are for example but not limited to, silica beads or membranes, magnetic beads,

### Use of a method or of a kit of the invention.

Aspects of the present invention relate to the use of any method or kit described herein.

In particular said use is the methylation-selective amplification of genomic DNA.
In particular, any herein described method or kit is preferably used for the sensitive detection of methylated or unmethylated DNA.

In particular, the use of a method or kit of the invention is preferred for at least one of the following with regard to a patient or individual: diagnosing a condition, prognosing a condition, predicting a treatment response, diagnosing a predisposition for a condition, diagnosing a progression of a condition, grading a condition, staging a condition, classification of a condition, characterization of a condition, or combinations thereof, wherein the condition is a healthy condition or an adverse event, the adverse event comprises at least one category selected from the group comprising: undesired drug interactions; cancer diseases, proliferative diseases or therewith associated diseases; CNS malfunctions; damage or disease; symptoms of aggression or behavioral disturbances; clinical; psychological and social consequences of brain damages; psychotic disturbances and personality disorders; dementia and/or associated syndromes; cardiovascular disease of the gastrointestinal tract; malfunction, damage or disease of the respiratory system; lesion, inflammation, infection, immunity and/or convalescence; malfunction, damage or disease of the body as an abnormality in the development process; malfunction, damage or disease of the skin, of the muscles, of the connective tissue or of the bones; endocrine and metabolic malfunction, damage or disease; and headaches or sexual malfunction.

Accordingly DNA is obtained from a sample derived from an individual or patient. Said nucleic acid is then analyzed according to the method of the invention. Preferably, therefore a kit of the invention is used. The results obtained by this analysis enable then the said diagnosing a condition, prognosing a condition, predicting a treatment response, diagnosing a predisposition for a condition, diagnosing a progression of a condition, grading a condition, staging a condition, classification of a condition, characterization of a condition, or combinations thereof.

In particular, the use of a method or kit of the invention is preferred for distinguishing cell types or tissue, or for investigating cell differentiation. Accordingly a nucleic acid is obtained ex vivo from samples of cells, cell types or tissue. Said nucleic acid is then analyzed according to the method of the invention. Preferably, therefore a kit of the invention is used. The results obtained by this analysis characterized the cells, cell types or tissue. Therewith the results enable to distinguish cell types or tissue, or the investigation of cell differentiation.

Aspects of the present invention relate to the use of the restriction enzyme Tsp509l or of one of its isochizomers for the analysis of cytosine methylation. Said isochizomers are for example but not limited to MiuCl, Sse9l, Tasl, TseCl, or TspEl. Preferably, Tsp509l or one of its isochizomers is applied according to the method of the invention for methylation-selective amplification or sensitive detection of methylation.

According to the invention, the herein disclosed restriction enzymes are replaced by one of their respective isochizomers.

All documents cited herein are incorporated by reference in their entirety.

### BRIEF DESCRIPTION OF THE FIGURES

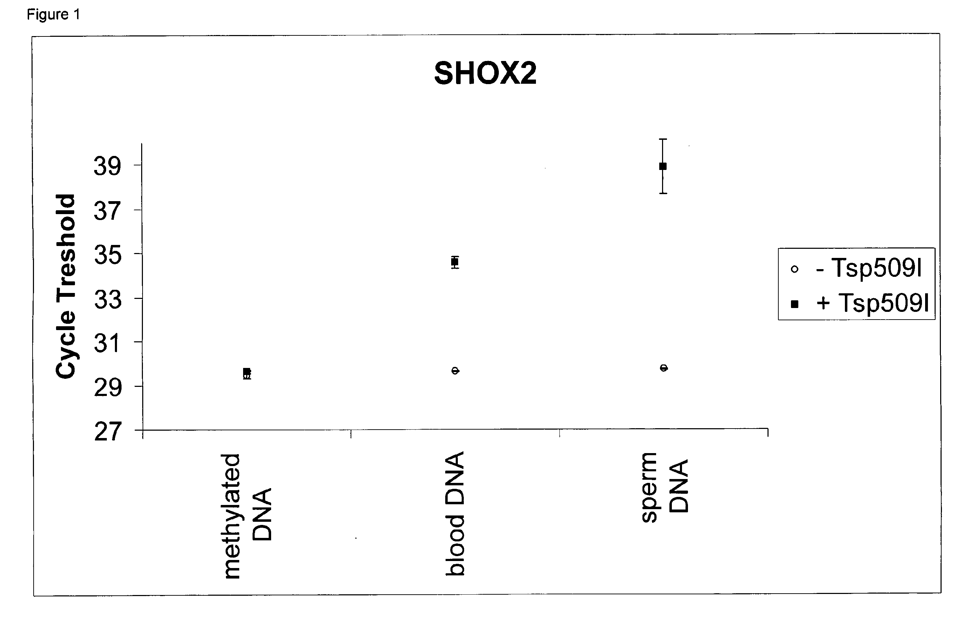

Figure 1: Cycle treshold resulted from the amplification of 5ng bisulfite converted DNA from sperm, blood and artificially methylated DNA. The amplification was done with and without Tsp509l restriction endonuclease. Each bar represents the mean of a triplicate measurement ± standard deviation. Bisulfite specific primers were used which specifically amplify a region within the SHOX2 gene.

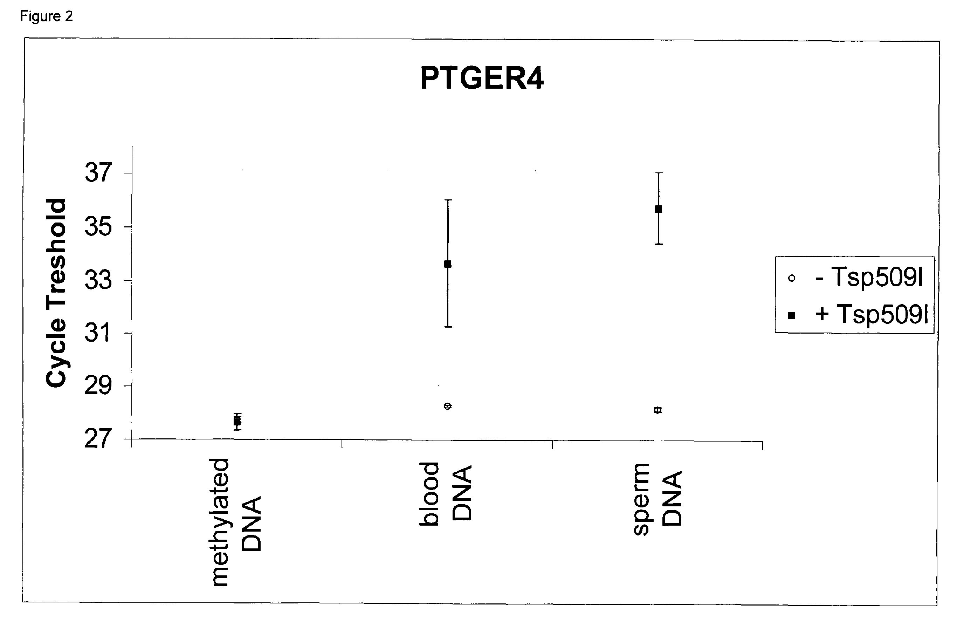

Figure 2: Cycle threshold resulted from the amplification of 5 ng bisulfite converted DNA from sperm, blood and artificially methylated DNA. The amplification was done with and without Tsp5091 restriction endonuclease. Each bar represents the mean of a triplicate measurement ± standard deviation. Bisulfite specific primers were used which specifically amplify a region within the PTGER4 gene.

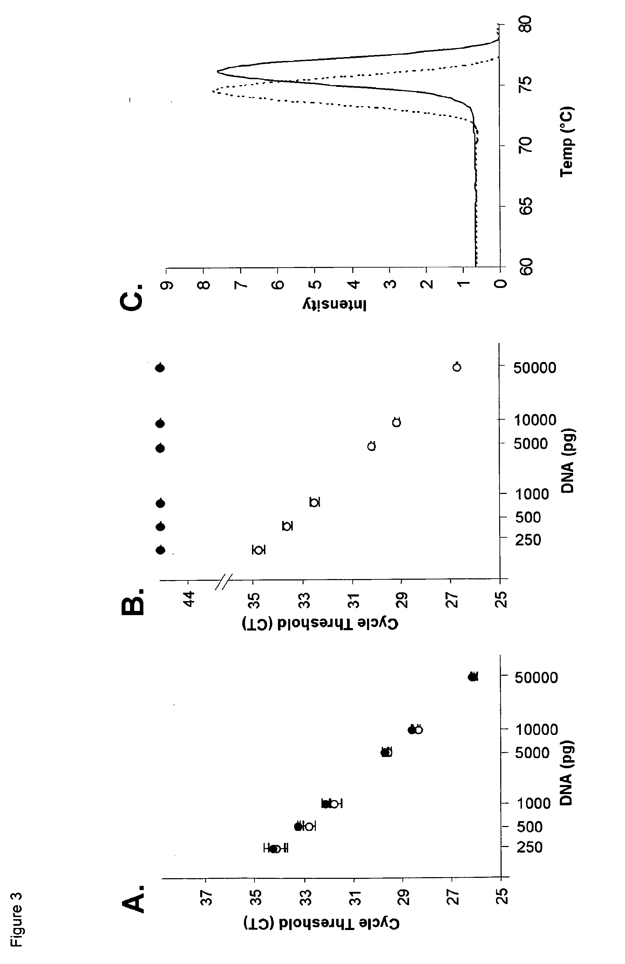

Figure 3: PCR efficiency in the presence of Tsp5091. Analysis of the BARLH2 locus in a dilution series of fully methylated (A) and unmethylated (B) template DNA, respectively, without (open circles) and with (closed circles) T*sp*509l enzyme. Shown are means of 4 (250, 500, 1000, 5000 pg) or three (10000 and 50000 pg) replicates ± standard deviation. Unmethylated and methylated DNA was amplified similarly in the absence of the enzyme (A).

Addition of the enzyme completely inhibits the PCR amplification of previously unmethylated DNA (B).
C: Resulting melting curves derived from methylated DNA (solid line) and unmethylated DNA (dotted line) in the absence of the enzyme. Shown are averaged curves of all single PCR reactions. PCR product derived from methylated DNA showed a higher melting temperature as compared to the unmethylated equivalent, due to the higher CG content.

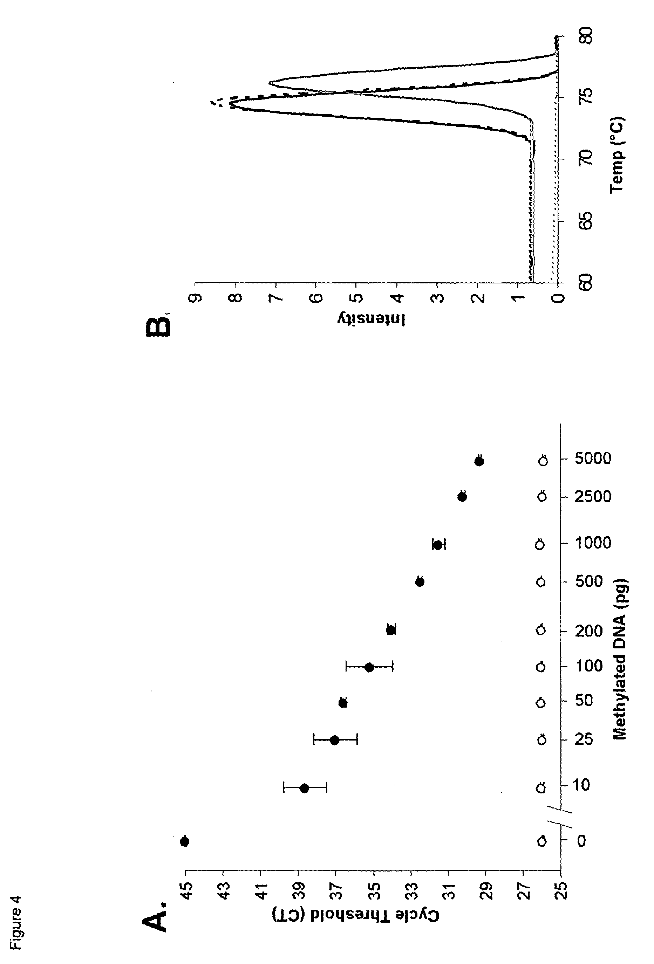

Figure 4: Sensitive detection of methylated DNA in a background of unmethylated DNA. A: Different amounts of methylated DNA were added to a constant background of 50 ng unmethylated DNA. PCR amplification was carried out in the absence (open circles) and presence (closed circles) of *Tsp*509l enzyme. Shown are means of 8 (0 pg and 10 pg methylated DNA in 50 ng unmethylated DNA), 4 (25, 50, 100 and 200 pg methylated DNA in 50 ng unmethylated DNA) or 3 replicates (500 and 1000 pg methylated DNA in 50 ng unmethylated DNA) replicates ± standard deviation, respectively. The CTs correlated with the spiked amounts of methylated DNA when *Tsp*509l was added. Without *Tsp*509l all samples showed low CTs due to the amplification of the high amount of background DNA. B: Melting curve analysis of the PCR product without (black lines) and with (grey lines) *Tsp*509l based restriction. Dotted lines represent the results of 50 ng unmethylated DNA without spiked methylated DNA (0 pg methylated DNA). Solid lines indicate the results of the mixtures containing methylated DNA (10 - 5000 pg methylated DNA). The depicted melting curves are averaged curves of all respective single melting curves. The melting curves confirmed the exclusive amplification on methylated DNA when *Tsp*509l is added down to 10 pg spiked methylated DNA, even in the presence of 5000 fold excess of unmethylated sequences.

### DEFINITIONS

In particular aspects, the term "methylation status" refers to, the presence or absence of methylation of the cytosine of a CpG positioin. Thereby said cytosine has the ability of being methylated or being non-methylated. A methylation status can be quantified, wherein it is considered over a plurality of molecules comprising said particular CpG position.

In particular aspects, the term "sensitive detection" refers to the detection of a single copy of a genome having a defined methylation status at a defined cytosine within 20, 50, 100, 1.000, 10.000, 100.000 or 1.000.000 genomic copies of background DNA. In case, the defined cytosine is methylated in the single genomic copy, the background genomic copies are unmethylated at the position of said cytosine. In case, the defined cytosine is unmethylated in the single genomic copy, the background genomic copies are methylated at the position of said cytosine. In particular preferred is the sensitive detection of a single human genomic DNA copy. A copy of the human genome has a weight of approximately 3,4 pg. Particularly preferred is the detection of a single DNA molecule having a defined methylation status at a defined cytosine within a background of 20, 50, 100, 1.000, 10.000, 100.000 or 1.000.000 DNA molecules. In case, the defined cytosine is methylated in the single DNA molecule, the background DNA molecules are unmethylated at the position of said cytosine. In case, the defined cytosine is unmethylated in the single DNA molecule, the background DNA molecules are methylated at the position of said cytosine.

All sequences herein are specified as reading from 5' to 3'.

The term "A" refers to adenine.

The term "T" refers to thymine.

The term "U" refers to uracil.

The term "G" refers to guanine.

The term "T/U" refers to thymine or uracil.

In particular aspects, the term "methylated DNA" refers to at least one CpG position in DNA being methylated. Accordingly in particular aspects, the term "unmethylated DNA" refers to at least one CpG position in DNA being unmethylated.

In particular aspects, the term "amplification product" refers to the resulting nucleic acid of an amplification. In particular, it refers to double stranded DNA resulting in the oligonucleotide or oligomer extending step of an amplification.

In particular aspects, the term "methylation-selective amplification" refers to target-oriented multiplication in dependence on the methylation status of at least one CpG position of a DNA molecule. Thus, in a methylation-selective amplification, either DNA is predominantly amplified that represents the case of methylated cytosine of said CpG positions or DNA is predominantly amplified that represents the case of unmethylated cytosine of said CpG positions. Preferably, in a methylation-selective amplification, either only DNA representing the case of methylated cytosine is amplified or only DNA representing the case of unmethylated cytosine is amplified.

### EXAMPLES

### Example 1: Methylation-selective amplification of regions of SHOX2 and PTGER4.

Experimental setup: Two real time PCR assays were carried out using bisulfite converted DNA derived from sperm, whole blood and artificially methylated DNA as template DNA. The assays are based on the amplification of regions within the SHOX2 and the PTGER4 loci, respectively. Both assays were carried out with and without Tsp5091 restriction endonuclease. DNA derived from sperm as well as DNA derived from whole blood are DNAs which show only low level methylation of the two analyzed loci.

Methods: DNA from washed research sperm (NW Andrology & Cryobank, Inc.) was extracted using the QlAamp DNA Mini Kit (Qiagen) according to the manufacturers' recommendations. Human genomic DNA isolated from whole blood was provided by Promega. Artificially methylated DNA (CpGenome™ Universal Methylated DNA) was supplied by Millipore. All DNAs were bisulfite converted using the EpiTect Kit (Qiagen) according to the handbook. DNA was quantified using the Nanodrop ND-1000 UV spectrophotometer (Nanodrop Technologies). 5 ng of each DNA type was applied in triplicates to each assay. The real time PCR were carried out in 20 µl volumes containing 0.3 µmol/l each primer, 0.5 µl of an 1000fold diluted SYBR Green (Biozym Scientific GmbH) dilution, 1 x QuantiTect Multiplex PCR NoROX kit (Qiagen), and optionally 1U Tsp5091 (New England Biolabs). Cycling was performed using a LightCycler LC480 (Roche) under the following conditions: 10 min at 95 °C and 45 cycles at 95 °C for 10 s, 56 °C for 30 s and 72°C for 10 s. Detection was done during the 56°C step. The following primers were used:

| | | |
|---|---|---|
| SHOX2-forward | SEQ ID NO: 1 | CCCTTCCTCTTTTTCTCTC |
| SHOX2-reverse | SEQ ID NO: 2 | GTTAATAATGAAAAAATGAGTAAAT |
| PTGER4-forward | SEQ ID NO: 3 | CAACCATACCTATTTCTACAAC |
| PTGER4-reverse | SEQ ID NO: 4 | TAGGTGTTTGGGTATTGTAGT |

Results: The cycle thresholds of the real time PCRs for the amplification of bisulfite DNA from sperm, whole blood and artificially methylated DNA are shown in Figure 1 and Figure 2. The amplification of all different DNAs without any added restriction enzyme resulted in comparable CTs. A addition of Tsp5091 endonuclease lead to an increase of the CTs for sperm DNA and DNA from whole blood as compared to artificially methylated DNA. The amplification of bisulfite converted artificially methylated DNA is unaffected. Melting curve analysis revealed that no originally unmethylated DNA was amplified in the presence of the enzyme. These results show that the addition of Tsp509l to the real time PCR efficiently suppresses the amplification of DNA which was originally not methylated at the respective CpG.

### Example 2: Highly sensitive and specific detection of methylated BARLH2 DNA using the heatstable restriction enzyme Tsp509l

### Abstract

DNA methylation is an important epigenetic mechanism involved in fundamental biological processes such as development, imprinting and carcinogenesis. Here, a new technique is presented for the detection of DNA methylation based on real-time PCR of bisulfite treated template with continuous enzymatic digestion of unmethylated DNA during amplification. To determine the analytical performance, the lung cancer methylation biomarker BARHL2 was analysed. The results demonstrate that the approach is useful for highly sensitive and specific detection of single methylated copies in a background of unmethylated DNA.

### Introduction

DNA methylation plays an important role in regulating cellular differentiation and development. As aberrant DNA methylation of specific loci is also linked to pathologic processes like carcinogenesis, the analysis of methylation-biomarkers represents a promising method for tumor diagnosis. The use of such biomarkers in body fluids, in particular in blood or urine, might especially allow for early detection of disease. However, these materials usually contain a high concentration of background DNA from normal cells and only a small portion of DNA from the tumor. Therefore, one major challenge for the application of methylation biomarkers in clinical diagnostic routine is the sensitive detection of single copies of methylated DNA in a high amount of unmethylated background DNA. Several methods exist which are applicable for DNA methylation analysis. However, most of these techniques do not allow for sensitive detection.
As of now, PCR analysis after bisulfite conversion is the method of choice when sensitive detection is desired. Bisulfite treatment of the template DNA leads to deamination of unmethylated cytosines to uracil, leaving only methylated cytosines unaltered, therefore transforming epigenetic information into sequence information. Methylation specific PCR (MSP) and HeavyMethyl (HM) assays are PCR based methods which allow for detection of single copies of methylated DNA. In MSP, primers bind specifically to previously methylated or unmethylated DNA, respectively, leading to specific amplification. Advantages of this technique are its high relative sensitivity, ease of design and low complexity of the reaction. However, for certain loci, primer design can be challenging as multiple CpGs have to be covered, preferably at the 3' primer end, to reach specificity.
Another technology with high sensitivity and specificity is the HM technology, a method based on amplification of bisulfite treated DNA using methylation unspecific primers in combination with methylation specific blocking oligonucleotides. In HM PCR, the high number of different oligonucleotides per assay can be challenging with respect to assay design. However, the possibility to adapt blocking conditions leads to increased flexibility and allows for the selective amplification of even such loci, where specific MSP is hardly possible. Methods, which allow for the sensitive detection especially at loci where MSP and HM assay design is challenging, would offer a significant improvement for DNA methylation analysis.

### Results

In this study, a new method for highly sensitive and specific detection of methylated DNA is introduced, which utilizes continuous cleavage of previously unmethylated DNA during the whole PCR process by the restriction endonuclease *Tsp*509l. *Tsp*509l is methylation insensitive and specifically cuts at AATT sites. The specific cleavage of unmethylated template is based on the presence of CpG dinucleotides in the sequence context 5'-AATCG-3' or 5'-AACCG-3' within the region of interest. After bisulfite conversion, this sequence will either be converted to 5'-AATCG-3' (CpG methylated) or 5'-AATTG-3' (CpG unmethylated), respectively. Therefore, only unmethylated CpG sites in the described sequence context result in a recognition site after bisulfite treatment, enabling the digestion of the template. The optimal incubation temperature of 65°C of the enzyme, its heat stability and high activity under different buffering conditions allow for the use of *Tsp*509l directly in the PCR reaction. The enzymatic activity leads to a continuous cleavage of previously unmethylated template during every PCR cycle, whereas methylated sequences remain unaffected and amplifiable. To determine the analytical performance of the *Tsp*509l assay, the lung cancer methylation biomarker BARHL2 (Bar like homeobox) was analyzed. Extraction of DNA from sperm (NW Andrology & Cryobank Inc., WA, USA) was performed using the QiaAmp^{®} DNA Mini Kit following the protocol optimized for sperm (Qiagen, Hilden, Germany). Bisulfite treatment of sperm DNA (unmethylated at the BARHL2 locus) and artificially methylated DNA (Millipore, MA, USA) was achieved as previously described (Tetzner R, Dietrich D, Distler J. 2007. Control of carry-over contamination for PCR-based DNA methylation quantification using bisulfite treated DNA. Nucleic Acids Res. 35: e4). PCR was carried out using the QuantiTect Multiplex Kit (Qiagen) with 0.3 µmol/l of each Primer:

| | | |
|---|---|---|
| BARLH2 forward | SEQ ID NO: 5 | acatataacaaatatattttatccaac |
| BARLH2 reverse | SEQ ID NO: 6 | attgtttgttagtttttaagttaat |

For methylation-specific conditions 1U *Tsp*509l (NEB, Ipswich, USA) was added to the reaction. Real time PCR was performed on the LC480 platform (Roche Diagnostics, Mannheim, Germany) with SYBR Green based detection and the following program settings: Initial denaturation 95°C, 15 min; 45 cycles of denaturation at 95°C, 10 s, annealing at 56°C for 30 s and detection at 72°C for 10 s. After 45 cycles, a melting curve analysis was performed.
Firstly, the PCR efficiencies were compared in the presence and absence of the restriction enzyme *Tsp*509l. Figure 3 shows that the presence of the enzyme does not impair the PCR efficiency since methylated template is amplified with the same efficiency, irrespective of the presence of *Tsp*509l. The PCR amplification of unmethylated DNA is completely inhibited in the presence of the enzyme *Tsp*509l while the amplification of methylated template is not impaired. Melting curve analysis confirmed the specific amplification of bisulfite DNA from methylated and unmethylated DNA, respectively.
To determine analytical performance in complex sample material, predefined mixtures of completely methylated and unmethylated DNA were used for analysis (Figure 4). When *Tsp*509l was added to the reaction, the methylated target was successfully amplified down to DNA concentrations of 10 pg (3 copies) in the presence of up to 5000-fold excess (50 ng) of unmethylated background DNA. The specific amplification of methylated DNA in a background of unmethylated DNA when adding *Tsp*509l was confirmed by melting curve analysis.

### Discussion

The usage of the restriction enzyme *Tsp*509l has been shown to be useful for highly sensitive and specific detection of methylated DNA in a background of unmethylated DNA. In general, DNA restriction is a common technique for methylation analysis. Methylation sensitive endonucleases have previously been used to digest non-bisulfite treated unmethylated template prior to PCR. The major disadvantage of this pre-PCR restriction is the fact that even minor remnants of non-digested DNA will be amplified leading to false positive results. However, such by-products are inevitable, as restriction enzymes do not reach complete digestion, especially when the DNA is fragmented or partially single stranded as expected in body fluids. Accordingly, the sensitive and specific detection of methylated DNA is limited. In COBRA (combined bisulfite restriction analysis), bisulfite-treated DNA is subjected to a single restriction step after PCR. In COBRA all DNA is amplified, without a preferred (biased) amplification of methylated DNA. Thus, samples which originally contained only traces of methylated DNA will also only contain minor portions of originally methylated DNA in the PCR product. These small amounts can hardly be detected via enzymatic restriction. The advantage of *Tsp*509l based cleavage during PCR is the resulting strong bias for the amplification of previously methylated DNA. The enzyme is active during the whole PCR leading to a continuous restriction of previously unmethylated (background) DNA, which compensates enzyme activities lower than 100%.
As the formation of a *Tsp*509l recognition site upon bisulfite treatment is a prerequisite for this technique, the method is only applicable to fragments which contain at least one AATCG or AACCG site. On the other hand, the bisulfite converted DNA must not contain the sequences AATT, AACC, AACT and AATC outside the CpG context. This would preclude analysis due to a complete cleavage of all DNA specimens, unless these sites are located in the primer binding site and can be mutated using respective mismatch primers.
The sequences required for the *Tsp*509l assay as described above are related to an analysis using the bisulfite converted leading strand as template. When using the bisulfite converted lagging strand for designing the assay, sequences containing the stretches, 5'-CGATT-3' or 5'-CGGTT-3' can also be analyzed. In this case, the sequences GGTT, AGTT, AATT and GATT outside the CpG context would preclude the analysis since these sequences would result in an AATT recognition site after bisulfite conversion.
In conclusion, an inexpensive and time saving procedure was developed for sensitive and specific amplification of methylated DNA which complements existing techniques quite well. Additionally, a combination of MSP and/or HM with *Tsp*509l cleavage is also a suitable tool for methylation analysis, especially in genomic regions with low CpG density or in regions where methylation specific priming or blocking sites are limited.

## Claims

1. Method for methylation-selective amplification, comprising
(a) subjecting a DNA sample to a treatment, wherein cytosine is converted to uracil, uracil sulfonate or another base having a different binding behavior than cytosine, while methylated cytosine remains unchanged; and
(b) amplifying the treated DNA by means of at least one oligonucleotide in the presence of an at least one restriction enzyme to generate an amplification product, wherein said restriction enzyme digests
- either the amplification product derived from converted methylated DNA,
- or the amplification product derived from converted unmethylated DNA during the amplification.

2. The method according to claim 1, wherein the at least one restriction enzyme is selected from the group consisting of Tsp509l, Acll, BstBl, BstUl, BstZ17l, CviQl, Hpal, and Tfil.

3. The method according to claim 1 or 2, wherein one of said restriction enzymes is an enzyme that is suitable for digesting the amplification product derived from unmethylated DNA, said enzyme having a recognition sequence that
(i) has a 5' terminal A, and does not comprise a G; and/or
(ii) has a 3' terminal T, and does not comprise a C.

4. The method according to claims 1 to 3, wherein one of said restriction enzymes is an enzyme said is suitable for digesting the amplification product derived from methylated DNA, said enzyme having a recognition sequence that comprises a cytosine of a CpG position.

5. The method according to claim 3, wherein the amplification product derived from treated unmethylated DNA comprise each at least one binding site (A) of said one or more oligonucleotides flanking an extended segment (B), wherein
- (A) and/or (B) comprise at least one sequence that either consist of the recognition sequence of the used enzyme and thereto an immediately 3' located G, or consist of the recognition sequence of the used enzyme and thereto an immediately 5' located C; and wherein
- (B) does not comprise a sequence being selected from the group consisting of recognition sequence of the used enzyme and thereto an immediately 3' located T, recognition sequence of the used enzyme and thereto an immediately 3' located A, recognition sequence of the used enzyme and thereto an immediately 3' located C, recognition sequence of the used enzyme and thereto an immediately 5' located G, recognition sequence of the used enzyme and thereto an immediately 5' located T, and recognition sequence of the used enzyme and thereto an immediately 5' located A

6. The method according to claim 5, wherein
(i) (A) comprises at least one sequence being selected from the group consisting of recognition sequence of the used enzyme and thereto an immediately 3' located T, recognition sequence of the used enzyme and thereto an immediately 3' located A, recognition sequence of the used enzyme and thereto an immediately 3' located C, recognition sequence of the used enzyme and thereto an immediately 5' located G, recognition sequence of the used enzyme and thereto an immediately 5' located T, and recognition sequence of the used enzyme and thereto an immediately 5' located A; and wherein
(ii) the treated DNA is amplified by means of at least one modified oligonucleotide.

7. The method according to claims 1 to 6, wherein the amplification product derived from treated methylated DNA comprise each at least one binding site (A) of said one or more oligonucleotides flanking an extended segment (B), wherein (A) and/or (B) comprise at least one recognition sequence of the used enzyme.

8. The method according to claim 1 to 7, wherein
(i) one of said restriction enzymes is Tsp509l; and
(ii) the amplification product derived from converted methylated DNA and the amplification product derived from converted unmethylated DNA comprise each at least one binding site (A) of said one or more oligonucleotides flanking an extended segment (B), wherein
- (A) and/or (B) comprise at least one sequence that corresponds to a sequence of a untreated DNA strand, said sequence being selected from the group consisting of AATCG, AACCG, CGATT, and CGGTT; and wherein
- (B) does not comprise a sequence corresponding to a sequence of an untreated DNA strand, said sequence being selected from the group consisting of AATT, AACT, AATCA, AATCT, AATCC, AACCA, AACCT, AACCC, AGTT, AATT, AGGTT, TGGTT, GGGTT, AGATT, TGATT, and GGATT.

9. The method according to claim 8, wherein
(i) (A) comprises at least one sequence that corresponds to a sequence of an untreated DNA strand, said sequence being selected from the group consisting of AATT, AACT, AATCA, AATCT, AATCC, AACCA, AACCT, AACCC, AGTT, AATT, AGGTT, TGGTT, GGGTT, AGATT, TGATT, and GGATT; and wherein
(ii) the treated DNA is amplified by means of at least one modified oligonucleotide.

10. The method according to claim 1 to 9, wherein
(i) one of said restriction enzymes is Acll or BstBl; and wherein
(ii) the amplification product comprises at least one sequence that corresponds to the sequence AACGTT of an untreated DNA strand.

11. The method according to claim 1 to 9, wherein
(i) one of said restriction enzymes is BstUl; and wherein
(ii) the amplification product comprises at least one sequence that corresponds to the sequence CGCG of an untreated DNA strand.

12. The method according to claim 1 to 9, wherein
(i) one of said restriction enzymes is BstZ171; and wherein
(ii) the amplification product comprises at least one sequence that corresponds to the sequence GTATAC of an untreated DNA strand.

13. The method according to claim 1 to 9, wherein
(i) one of said restriction enzymes is CviQl; and wherein
(ii) the amplification product comprises at least one sequence that corresponds to the sequence GTAC of an untreated DNA strand.

14. The method according to claim 1 to 9, wherein
(i) one of said restriction enzymes is Hpal; and wherein
(ii) the amplification product comprises at least one sequence that corresponds to the sequence GTTAAC of an untreated DNA strand.

15. The method according to claim 1 to 9, wherein
(i) one of said restriction enzymes is Tfil; and wherein
(ii) the amplification product comprises at least one sequence that corresponds to the sequence GAWTC of an untreated DNA strand.

16. The method of claim 1 to 15, additionally comprising quantifying the undigested amplification product of step (c) during the amplification or thereafter.

17. The method of claim 16, wherein
(i)
- amplification product derived from treated unmethylated DNA is digested in step (c) of claim 1, and wherein
- the amount of methylated DNA in the DNA subjected in step (a) of claim 1 is deduced from the results of the quantification of undigested amplification product; or wherein
(ii)
- amplification product derived from treated methylated DNA is digested in step (c) of claim 1, and wherein
- the amount of unmethylated DNA in the DNA subjected in step (a) is deduced from the results of the quantification of undigested amplification product.

18. A kit, comprising
(a) a bisulfite or a cytidin-deaminase;
(b) at least one oligonucleotide or oligomer usable as a primer;
(c) a polymerase and/or a ligase;
(d) at least one enzyme of the group consisting of Tsp509l, Acll, BstBl, BstUl, BstZ17l, CviQl, Hpal, and Tfil; and
(e) optionally, at least one selected from the group consisting of ethidium bromide, SYBR green, probe, oligonucleotide probe, PNA probe, Taqman probe, Lightcycler probe, scorpion primer, RNAenzyme, DNAenzyme, blocker, blocker oligonucleotide, blocker PNA oligomer.

19. Use of a method or of a kit of a preceding claim for the sensitive detection of methylated or unmethylated DNA.

20. Use of a restriction enzyme selected from the group consisting of Tsp5091, MluCl, Sse91, Tasl, TseCl, TspEl, and an isochizomer of Tsp5091 for the analysis of cytosine methylation.


Sequence listing

<110> Epigenomics AG

<120> METHOD FOR METHYLATION-SELECTIVE AMPLIFICATION

<130> FBP21660

<160> 6

<210> 1
<211> 19
<212> DNA
<213> Artificial Sequence

<220>
<223> chemically treated genomic DNA (Homo sapiens)

<400> 1

cccttcctct ttttctctc                                                    19

<210> 2
<211> 25
<212> DNA
<213> Artificial Sequence

<220>
<223> chemically treated genomic DNA (Homo sapiens)

<400> 2

gttaataatg aaaaaatgag taaat                                             25

<210> 3
<211> 22
<212> DNA
<213> Artificial Sequence

<220>
<223> chemically treated genomic DNA (Homo sapiens)

<400> 3

caaccatacc tatttctaca ac                                                22

<210> 4
<211> 21
<212> DNA
<213> Artificial Sequence

<220>
<223> chemically treated genomic DNA (Homo sapiens)

<400> 4

taggtgtttg ggtattgtag t 21

<210> 5
<211> 27
<212> DNA
<213> Artificial Sequence

<220>
<223> chemically treated genomic DNA (Homo sapiens)

<400> 5

acatataaca aatatatttt atccaac 27

<210> 6
<211> 25
<212> DNA
<213> Artificial Sequence

<220>
<223> chemically treated genomic DNA (Homo sapiens)

<400> 6

attgtttgtt agtttttaag ttaat